# EUROPEAN PATENT APPLICATION

(11) **EP 4 710 988 A1**
(43) Date of publication of application: **18.03.2026**
(21) Application number: 24200616.1
(22) Date of filing: 16.09.2024
(51) Int. Cl.: A61M 37/00

(54) **APPLICATION DEVICE FOR MICRONEEDLES, MICRONEEDLE APPLICATION SYSTEM AND METHOD FOR MANUFACTURING A MICRONEEDLE APPLICATION SYSTEM**

(71) Applicant: LTS LOHMANN Therapie-Systeme AG, 56626 Andernach (DE)
(72) Inventor: Kuch, Christopher, 56410 Montabaur (DE); Scherr, Sebastian, 56335 Neuhäusel (DE); Heidary Dastjerdi, Dr. Maral, 53424 Remagen (DE)
(74) Representative: dompatent

(57) **Abstract**

Application device (10) for microneedles, comprising: a housing (16) comprising a base (12) adapted to be arranged on the skin, a punch (18) arranged inside the housing (16), wherein the punch (18) is movable inside the housing (16) in an application direction (11) from a starting position (A) to an application position (B) to apply an application force (112) to a microneedle array (102) for applying the microneedle array (102) into the skin, wherein in the starting position (A) the punch (18) is secured against movement in the direction opposite to the application direction (11). Further disclosed are a microneedle application system (100) and a method for manufacturing a microneedle application system.

## Description

The present invention relates to an application device for microneedles, a microneedle application system and a method for manufacturing a microneedle application system.

Microneedles are used to deliver active ingredients directly into the skin. For this purpose, the microneedles are just long enough to penetrate only the outer skin layers, but preferably do not reach nerves and blood vessels. Generally, microneedles have a length of 25 to 2000 µm.

A plurality of the microneedles is usually assembled as a microneedle array, whereby the microneedles are arranged on a backing element, such as a patch, plaster, or plate. Particularly, the microneedles are detachably or non-detachably connected to the backing, or the microneedles are integrally formed with the backing. Microarrays often have a high number of microneedles, for example, 100 to 600 microneedles per cm².

One concept for delivering active ingredients using microneedles is to coat, for example, solid and/or non-dissolvable microneedles with the active ingredient. Upon insertion of the microneedle into the skin, the active ingredient can be released, for example, dissolved, from the microneedle and thus be delivered into the skin.

Furthermore, microneedles of the state of the art can be made of fully or partially dissolvable materials containing the active ingredient. For example, biocompatible polymers are used in dissolving microneedles, which form the matrix for the active ingredient. Once the microneedles have penetrated the skin, they dissolve and release the active ingredient into the skin. The polymer, which is also absorbed, breaks down in the body and is excreted. After insertion, the microneedles can be applied while being connected to the backing or, after insertion, the needles can be detached, for example, broken off, from the backing.

Dissolvable microneedles can have multiple layers, particularly comprising different materials per layer. For example, the tip of the microneedle can comprise the active ingredient, while the rest of the microneedle is free of active ingredient. Furthermore, if the backing is integrally formed with the microneedles, the backing, particularly integrally together with a stump of each of the microneedles, can be made of a material different, preferably free of active ingredient, to the material of at least the tip of the microneedles.

The correct positioning of the microneedle array on the skin and/or the correct application of the microneedles into the skin is essential for microneedle administration. To achieve positioning and/or application, application devices, i.e. applicators, for the microneedle arrays are known in the art.

Generally, such applicators comprise complex mechanisms for ensuring the right positioning and application of microneedle array. The applicators are often devices having bulky and/or heavy housings. Furthermore, the devices are generally adapted for use in medical practices or clinics. Known applicators are very costly to produce and should often only be used by skilled medical staff. Applicators are often meant to be used multiple times, which requires sterilization of them at certain intervals. On the one hand, this makes access to microneedle arrays more difficult, especially in countries that already have challenges with medical care. On the other hand, it is not possible for the patient to use the microneedle array discretely and/or on his own.

Additional issues with known applicators are incorrect or no penetration of at least some of the microneedles per microneedle array as well as incorrect application, for example force application, particularly over a certain time.

Furthermore, existing manufacturing methods for manufacturing microneedle array systems are complex and expensive, for example due to manual manufacturing steps.

Thus, it is an object of the present invention to provide an optimized application device for microneedles, an optimized microneedle application system and an optimized method for manufacturing a microneedle application system.

The object is solved by an application device for microneedles according to claim 1, a microneedle application system according to claim 11 and a method for manufacturing a microneedle application system according to claim 12.

The present invention relates to an application device for microneedles, particularly for microneedle arrays. The application device comprises a housing comprising a base. The base, particularly a skin surface of the base, is adapted to be arranged on the skin, preferably of a person and/or an animal. A, preferably cylindrical, punch is arranged, particularly at least partially or completely, inside the housing, preferably inside the base. For example, the punch can be arranged in a, preferably cylindrical, opening of the housing, particularly of the base. The housing, particularly the base, preferably has an application opening on the skin surface for moving the punch, particularly partially, out of the housing. The punch is arranged movable inside the housing, particularly the base, in an application direction from a starting position to an application position to apply an application force to a microneedle array for applying the microneedle array into the skin. Preferably the application force is in range of 50 N and 200 N, more preferably in range of 120 N and 140 N. The punch is preferably movable relative to the housing, particularly the base. Preferably the punch is arranged linearly movable and/or slidable inside the housing. The application position particularly corresponds to a final position, whereby it is preferred that the punch is in a locked state. In the starting position the punch is preferably secured against movement in the direction opposite to the application direction. However, the present invention is also directed to the application device, wherein the feature that the punch is secured against movement in the direction opposite to the application direction is optional. Secured against movement preferably means that the punch is in a locked and/or engaged state, particularly locked and/or engaged with the housing, preferably the base. It is preferred that the punch comprises a microneedle surface that is connectable or connected to the microneedle array. The punch may comprise a step having a smaller cross-section and/or diameter than the outer wall, particularly the cylindrical surface, of the punch, wherein the step comprises the microneedle surface. Preferably, the housing provides a sterile packaging, particularly for the punch and/or for a microneedle array connected to the punch.

Preferably, the application device comprises at least one latching device between the housing, particularly the base, and the punch to secure the punch in the starting position and/or in the application position against a movement in the direction opposite to the application direction. The latching device is preferably connected to the housing and/or the punch, particularly integrally connected. For example, the latching device may comprise a first latching element connected to the housing and/or the latching device may comprise a second latching element connected to the punch. The first and second latching elements preferably interact, particularly latch, with each other. The latching device is particularly a form-fit and/or force-fit latching device. Particularly, the latching device, for example the first and/or second latching element can be arranged radially to the housing and/or the punch. It is preferred that the application device comprises at least one latching device to secure the punch in the starting position against a movement in the direction opposite to the application direction and/or at least one latching device to secure the punch in the application position against a movement in the direction opposite to the application direction. The first and/or the second latching element is preferably deflectable. It is preferred that the first latching element is arranged radially in the opening of the housing, particularly the base. The second latching element is preferably arranged, particularly radially, on an outer wall, for example a shell surface, of the punch.

Preferably, the latching device comprises a projection, an edge, a groove, a snap and/or cantilever hook, a tab, and/or a protrusion. For example, the first latching element can be a groove or an edge and/or the second latching element can be a hook, a tab or a projection, whereby the second latching element interacts, particularly latches, with the first latching element. On the other hand, for one or more of the latching devices, the second latching element can be a groove or an edge and/or the first latching element can be a hook, a tab or a projection.

Preferably, the latching device comprises a one-way lock to prohibit movement of the punch in the direction opposite to the application direction. Particularly, the one-way lock is adapted such that if the punch is moved in the application direction, the lock, for example a stop surface of the one-way lock, is overcome, particularly passed, whereupon the punch is immovable back in the direction opposite to the application direction. The one-way lock is preferably adapted to prohibit the movement of the punch from the starting and/or the application position.

Preferably, the latching device is adapted to allow free, in particular unimpeded, movement, for example sliding movement, of the punch from the starting position and/or the application position in the application direction. Particularly, the free movement of the punch in the application direction is implemented such that the punch is arranged movable free of form-fit and/or free of force-fit within the housing.

Preferably, the application device comprises an, preferably adhesive, patch to attach the base to the skin. The patch is particularly connected to the housing. The patch is preferably stretchable. It is preferred that the patch comprises an opening, particularly in the center of the patch to accommodate the housing, in particular in such a way that the patch surrounds the housing. Preferably, the patch, particularly in the area of the opening, is flanged between the cover and the base. The patch is particularly clamped between the cover and the base. Particularly, a cross-sectional area of the patch, in particular a surface area, is at least double, preferably at least triple, more preferably at least four times as large as a cross-sectional area of the housing. The patch particularly has a cross-sectional area, in particular a surface area, of 1000 mm² to 25000 mm², preferably of 1500 mm² to 10000 mm², more preferably of 2000 mm² to 6000 mm².

Preferably, the patch is adapted to tension the base against the skin, in particular against a restoring force exerted via the punch. The restoring force corresponds to the force exerted from the skin against the punch, particularly against the application force. The patch therefore preferably provides a tension force driving the application device, particularly at least the punch, onto the skin. It is preferred that the patch is connected to the housing in a distance to the skin surface of the base. The distance of the patch attached to the housing to the skin surface is preferably 1 mm to 10 mm, more preferably 2 mm to 5 mm. Due to the distance, it is preferred the tension is provided. The tension is preferably exerted by stretching the patch, more preferably by a reset force acting against the stretching of the patch. The tension particularly provides a further, preferably lasting, pressing of the punch, such that the microneedle array connected to the punch can be applied over a time period with pressure.

Preferably, the housing comprises a cover, for example a lid, connected to the base closing the base on a side opposite a skin surface of the base. The cover and the base are preferably connected by a form-fit and/or materially bonded, for example adhesively. For example, the cover and the base can be connected by a pin-connection, whereby the cover and/or the housing comprises one or more pins to be connected into pin receptacles of the other of the cover and the housing.

Preferably, the cover encloses the punch within the housing, in particular to prevent removal of the punch from the housing on the side of the cover. The cover particularly prevents removal of the punch in the direction opposite the application direction.

Preferably, the patch is fixed, particularly pierced, between the cover and the base. For example, the patch can be fixed by the pins, particularly by piercing the pins through the patch. The pins can be spikes. The patch may comprise pre-cut openings to insert the pins and/or openings can be pierced into the patch by the pins.

It is preferred that the patch is folded in an initial state, in particular a delivery state. It is particularly preferred that the patch is folded against the housing, e.g. the base or the cover. For example, the patch can be folded on the area of the skin surface of the base or on a surface of the cover. In particular, the patch has two folds, whereby preferably two wings of the patch are folded each. Preferably adhesive surfaces of the patch are folded, such that the adhesives are covered in the initial state. The patch can be unfolded for use.

It is preferred that the housing, preferably the base, has a diameter of 10 mm to 100 mm, preferably of 20 mm to 60 mm. The opening through the housing, particularly the base, has particularly a diameter of 5 mm to 70 mm, preferably of 10 mm to 30 mm. The punch preferably has a diameter of 5 mm to 70 mm, preferably of 10 mm to 30 mm. Particularly the punch has a smaller diameter than the opening through the housing, particularly the base, for example smaller in the range of 0,1 mm to 1 mm, preferably 0,2 mm to 0,6 mm.

Preferably, the application device comprises an operation device, particularly a lever or a button, acting on the punch to transfer a force to the punch corresponding to the application force. The operation device, particularly the application device, is free of energy storage. Particularly, the punch is not moved by a spring. The operation device is preferably adapted to be operated by a user, particularly the hand of a user. The operation device thus preferably comprises an element, for example a surface, on which a user can exert an operational force to be transferred to the punch. Preferably, the operation device is directly connected and/or directly transfers the force to the punch. If the operation device comprises or is a lever, it is preferred that one end of the lever is adapted for actuation by the user and the other end is adapted to directly contact and as such displace the punch. In particular, the lever can be folded from one side to the other, whereby particularly the lever deflects the plunger to the maximum in a central position. If the operation device comprises or is a button, the button preferably corresponds to a, particularly round, cylinder. It is preferred that one side, particularly one surface, of the button is adapted for actuation by the user and the other side, particularly surface, is adapted to directly contact and as such displace the punch. The button is preferably movable linearly. It is preferred that the operation device is adapted such that the operational force acting on the operation device by a user corresponds or correlates to the force transferred to the punch. The application device may be connected to the housing and/or the punch. The connection between the applicator device and the housing and/or the punch is implemented, for example, through a form-fit and/or force-fit; or integrally. In a preferred embodiment in which the application device comprises a lever, a lever mount, for example a pivot joint, for the lever can be connected to the housing, for example the cover, particularly integrally. In another preferred embodiment in which the application device comprises, particularly consist of, a button, the button is preferably directly connected to the punch, for example via form-fit and/or force-fit and/or materially bonded. The button can be arranged movable within the housing, for example movable inside the base, particularly coaxially with the punch.

Preferably, the application device comprises a visual marking visible, particularly exposed, when the punch has been displaced from the starting position, preferably into the application position. The marking preferably comprises a color, for example a colored surface, and/or a symbol, and/or a text. It is preferred that the marking is only visible when the punch has been displaced. The color is preferably green.

Preferably, the visual marking is exposed by a movement of the operation device. Particularly, the visual marking is arranged on the operation device and/or is covered by the operation device in the starting position. The marking is, for example, arranged on a surface of the lever which is concealed in a starting position of the lever and exposed in an application position of the lever. On the other hand, if the application device comprises a button, the visual marking can be arranged on the button or on a guide of the button, for example the housing, particularly the base. Upon movement of the button from a starting position to an application position, the visual marking can become visible, for example if the moving button exposes the marking on the housing or if the marking on the button is moved to an exposed area, e.g. a window in the housing.

Preferably, in the starting position, the punch and/or the operation device, particularly the button, is arranged flush with a top surface of the housing, preferably the cover, or set back inwards to the top surface. The top surface is preferably arranged opposite to the skin surface and/or opposite to the application opening of the housing. Alternatively, the punch and/or the operation device can be arranged protruding from the top surface in the starting position. It is preferred that the punch and/or the operation device is arranged flush with the top surface or set back inwards to the top surface in the application position.

Preferably, the application device provides the user with a response regarding successful application of the microneedle array into the skin. The response might include an audible, visible and/or tactile signal. It is conceivable, the application device, particularly the operation device, is adapted to provide the visible and/or tactile signal when the punch is moved in the application direction from the starting position to the application position and the microneedle array is applied into the skin. Preferably, the response, in particular visible and/or tactile signal, is provided to the user when the punch reaches the application position. For example, if the button is flush with the top surface of the housing in the starting position and, after applying the application force, set back inwards to the top surface in the application position, an inner lateral surface of the housing is visible. Alternatively, if the button protrudes of the top surface of the housing in the starting position and a lateral surface of the button is visible, after applying the application force, the top surface is flush with the button and the lateral surface of the button is not visible. It is conceivable that the inner lateral surface of the housing or the lateral surface of the button is indicated by color and/or comprises the visual marking. Preferably, the application device, particularly the latching device, is adapted to provide the audible and/or tactile signal when the punch reaches the application position. The audible and/or tactile signal can be emitted by the latching device. For example, if the latching element snaps due to deflection and/or snaps back from deflection, the latching element may emit an audible and/or tactile signal.

Preferably, the housing, particularly the base, comprises an adhesive to be adhered to the skin. The adhesive is preferably arranged on the skin surface. With this adhesive, the application device can be positioned on the skin, particularly initially positioned. Preferably after the initial positioning, the application device can be fixed to the skin by the, particularly adhesive, patch.

The invention further relates to a microneedle application system, comprising the application device according to the invention. The system further comprises a microneedle array. The microneedle array is connected or connectable to the punch. Preferably, the microneedle array is connected to the punch, particularly the microneedle surface, via a material-bonded, for example an adhesive, connection and/or a form-fit; or integrally. If the microneedle array is connected integrally with the punch, it is preferred that the microneedle array and the punch are molded together, for example in the same molding form. Preferably, the microneedle array is connected to the punch in the starting position and/or the application position. The microneedle array comprises a plurality of microneedles, for example 100 to 600 microneedles per cm². It is preferred that the microneedles are particularly partially or fully dissolvable microneedles, or coated microneedles. The microneedles, preferably the tip of the microneedles, comprises an active ingredient. Particularly, the microneedles have a length of 25 µm to 2000 µm, preferably of 50 µm to 1200 µm. The microneedle array may comprise a backing, such as a patch, plaster or plate, or the microneedle array may consist of individual separate microneedles. If the microneedle array consists of individual separate microneedles, it is preferred that after connecting the punch to the microneedle array, the punch corresponds to the backing of the microneedle array. The application system can comprise a package, e.g. a wrapper, for sterile packaging of the application device and in particular the microneedle array. It is preferred that the microneedle array, particularly the backing, has a diameter of 5 mm to 70 mm, preferably 8 mm to 25 mm. Preferably, the microneedle array, particularly the backing, has a smaller diameter than the punch and/or the opening through the housing, particularly the base, for example smaller in the range of 0,5 mm to 5 mm, preferably 1 mm to 3 mm.

For the microneedle application device and/or the microneedle application system, it is preferred that the microneedle surface of the punch and/or the microneedle array, particularly tips of the microneedles of the microneedle array, are arranged: flush with the skin surface of the base; set back from the skin surface within the housing; or protruding from the skin surface outside the housing.

The invention further relates to a method for manufacturing a microneedle application system, preferably the microneedle application system according to the invention. The method comprises the steps of: i. manufacturing of a microneedle array in a mold; ii. providing, preferably manufacturing, more preferably molding, a housing and a punch; and iv. arranging the punch movable inside the housing, particularly inside a base of the housing. Preferably steps i. and ii. are carried out simultaneously or one after another. Step iv. is particularly carried out after step i. and/or ii.

Preferably, the method comprises the further step of: iii. demolding the microneedle array from the mold by the punch. Step iii. is preferably carried out after step i. and/or before step iv. In order to demold the microneedle array by the punch, it is preferred to adhesively connect the punch to the microneedle array. For example, the punch may be brought into contact with the microneedle array, particularly with a backing or separate individual microneedles of the microneedle array, e.g. by pressing it onto the microneedle array. An adhesive may be arranged between the punch and the microneedle array. The punch can comprise a bulge, particularly on the microneedle surface to demold the microneedle array with the bulge. On the other hand, it is preferably possible that when bringing the punch into contact with the microneedle array, the microneedle array, particularly a backing or separate individual microneedles of the microneedle array, is not fully dried yet thereby adhering the punch to the microneedle array. Another preferred example to demold the microneedle array by the punch is by molding the punch in direct contact with the microneedle array, particularly a backing or separate individual microneedles of the microneedle array, for example rearwards to the microneedle array. Particularly, the punch may be molded in the same mold as the microneedle array, for example in a cavity of the mold connected to the cavities for the microneedles or a cavity for the backing. As such, it is preferred that the punch corresponds to the backing of the microneedle array.

Preferably, the method comprises the further step of connecting a patch to the housing, particularly arranging the patch between a base and a cover of the housing. In particular, the base and the cover are connected to each other, e.g. flanged, whereby it is preferable that the patch is clamped between them. It is preferred that connection pins of the base are connected to pin openings of the cover, and/or vice versa, whereby it is preferred that the patch is pierced by the pins and as such fixed to the housing, particularly between the cover and the base. The step of connecting the patch to the housing is preferably carried out before or after step iv.

Preferably, the step of arranging the punch movable inside the housing comprises: inserting, particularly sliding, the punch into the housing, particularly the base, into a starting position by passing the punch over a latching device between the housing and the punch to secure the punch in the starting position against a movement in the direction opposite to an application direction of the movement of the punch.

In the following the present invention is described in more detail with reference to the accompanying drawings.

It is preferred that one or more objects according to the invention, i.e. the application device, the application system and/or the method, comprise one or more features of the other objects according to the invention.

The figures show:
- Figure 1: a sectional side view of an embodiment of an application system comprising an embodiment of an application device, in a first state;
- Figure 2: the application system of Fig. 1 in a perspective view;
- Figure 3: the application system of Fig. 1 in an exploded view;
- Figure 4: the application system of Fig. 1 in a second state;
- Figure 5: an exploded sectional view of another embodiment of an application device; and
- Figures 6a-6b: sectional side views of another embodiment of an application system comprising another embodiment of an application device, in a first and second state.

Similar or identical elements are identified in the figures with the same reference signs. For improved clarity, in particular elements that have already been identified are generally not given reference signs in all figures.

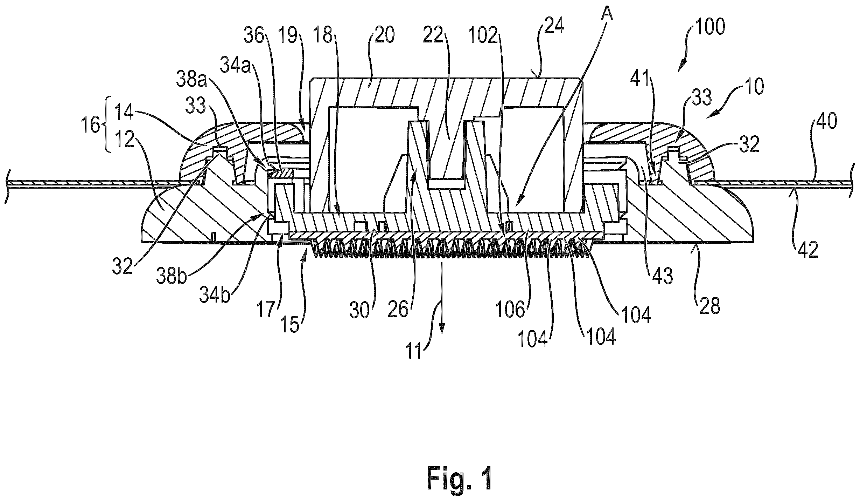

Figure 1 shows a preferred embodiment of the application system 100 according to the invention comprising a preferred embodiment of the application device 10 according to the invention.

The application device 10 comprises a housing 16 comprising a base 12 and a cover 14 connected, particularly flanged, to the base 12. The connection between the base 12 and the cover 14 is achieved by a pin connection, whereby the base 12 has a plurality of, in the shown embodiment four, pins 32 which are connected to pin openings 33 of the cover, particularly by a form-fit and/or force fit.

The base 12, particularly a skin surface 28 of the base, is preferably adapted to be arranged on a skin of a user (not shown). The skin surface 28 can comprise an adhesive to adhesively position the base 12 on the skin.

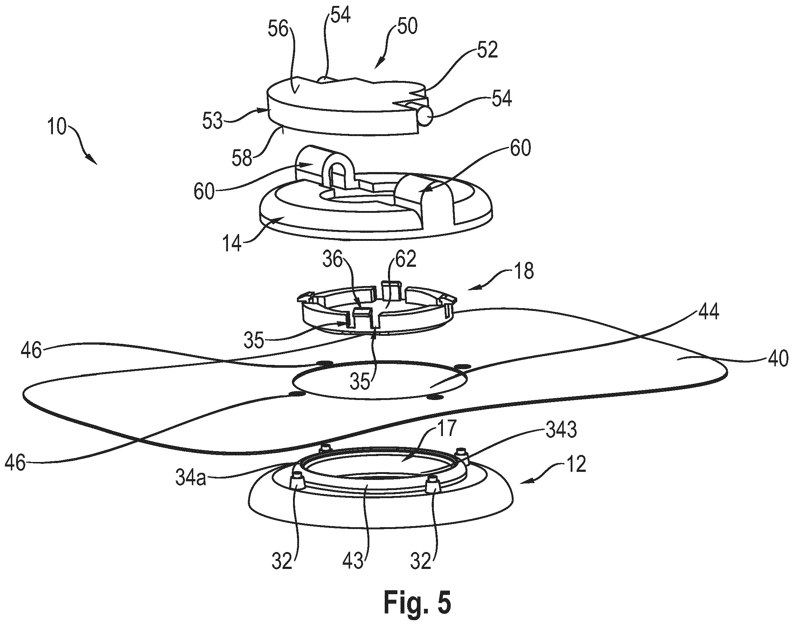

A patch 40 is arranged, particularly clamped, between the base 12 and the cover 14 and as such connected to the housing 16. The patch 40 has a central opening 44 (see Fig. 5) abutted on a ridge 43 of the base 12 to be positioned and form-fitted. The patch 40 can have pre-cut pin openings 46 (see Fig. 5) to pass the pins 32 therethrough or the openings 46 can be pierced through the patch 40 by the pins upon mounting the cover 14 to the base 12. The patch 40 is preferably flexible and/or stretchable.

Additionally, the cover 14 and the base 12 can be connected adhesively, for example by an adhesive (not shown) between the pins 32 and the pin opening 33 and/or by an adhesive (not shown) between the base 12 and the patch 40 and between the patch 40 and the cover 14.

The housing 16 has a central, preferably cylindrical, opening 15 formed by an opening 17 through the base 12 and an opening 19 through the cover 14.

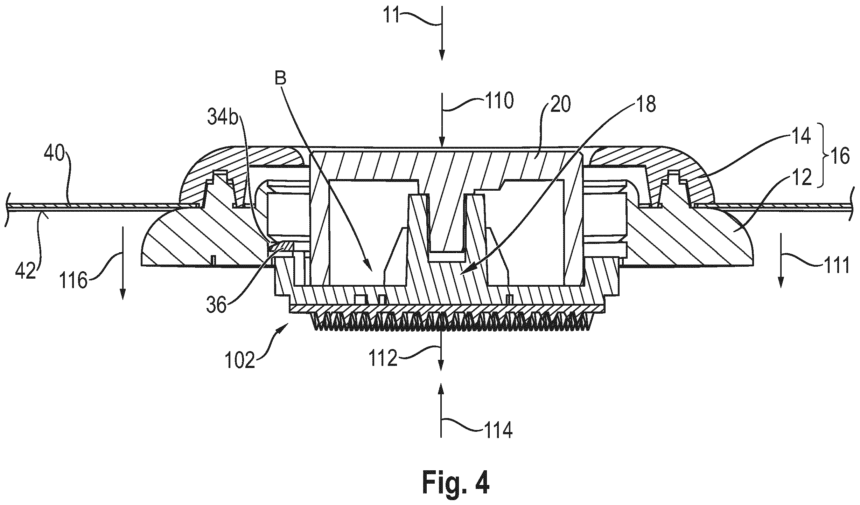

A punch 18 is arranged movable within the opening 17 of the base 12 between a starting position A shown in Fig. 1 and an application position B (shown in Fig. 4).

The application device 10 comprises a latching device 38a between the base 12 and the punch 18 to secure the punch in the starting position A against a movement in the direction opposite to the application direction (the application direction is indicated by arrow 11) and a latching device 38b between the base 12 and the punch 18 to secure the punch in the application position B against a movement in the direction opposite to the application direction 11. The latching devices 38a, 38b are implemented by deflectable hooks 36 radially connected, particularly integrally, to the punch 18 and corresponding radial protrusion 34a, 34b connected, particularly integrally, to the base 12 arranged in the opening 17. The protrusions 34a, 34b are preferably arranged circumferential, particularly fully circumferential, and for example correspond to projections and/or edges in the opening 17.

It is preferred that when manufacturing the application device 10, the punch 18 is inserted into the opening 17, whereby the hooks 36 deflect to pass the protrusion 34a, thereby arranging the punch 18 in the starting position A (as shown in Fig. 1). In this position, the protrusion 34a provides a stop surface for the hooks 36 and as such prohibit movement of the punch in the direction opposite to the application direction 11. On the other hand, from the starting position A, the punch 18 is free to move in the application direction. Particularly, there is no form-fit influencing movement of the punch 18 from the starting position A.

Upon movement of the punch 18 from the starting position A due to an application actuation of the application device 10, the hooks 36 again deflect to pass the protrusion 34b, thereby arranging the punch 18 in the application position B (as shown in Fig. 4). In this position, the protrusion 34b now provides a stop surface for the hooks 36 and as such prohibit movement of the punch in the direction opposite to the application direction 11, particularly back into the starting position A. Preferably, the protrusions 34a, 34b are unpassable by hooks 36 in the direction opposite to the application direction 11. The protrusions 34a, 34b therefore preferably correspond to one-way locks.

Deflection of the hooks 36 is preferably achieved by a radial movability of the hooks 36, particularly implemented by a flexible connection of the hooks 36 to the punch 18, for example due to the cut outs 35, which allow for an individual implementation and thus flexibility of the hooks 36 (see Fig. 5).

In the embodiment shown in Fig. 1, the application system 100 and the application device 10 are in a first state, corresponding to starting a state, in which the punch 18 is in the starting position A (Fig. 4 shows the second state).

A microneedle array 102 comprising a backing 106 holding a plurality of microneedles 104 is connected with the backing 106, particularly via an adhesive, to a microneedle surface 30 of the punch 18. In the shown embodiment, the microneedle array 102 comprises a separate backing 106. However, in a (not shown) preferred alternative embodiment the microneedles can be directly, for example integrally, connected to the punch 18.

The punch 18 is movable, particularly displaceable, by an operation device, which in the embodiment of Fig. 1 corresponds to a button 20. Upon actuation, in particular by pressing the button 20 on the application surface 24 by a user, for example the hand, particularly the finger, of the user, the button 20 is displaced linearly in the application direction 11. The button 20 is connected to the punch 18 by a form-fit and/or force-fit connection implemented by a connection between a pin 22 of the button 20, which is fitted into a pin holder 26 of the punch 18. Thus, upon actuation, i.e. pressing, of the button 20, the pressing force 110 (see Fig. 4), particularly of the user, is transferred to the button 20 and from the button to the microneedle array 102. In view of this, the microneedle array 102 is moved and pressed and as such can be applied into a skin (see Fig. 4). The pressing force 110 of the user preferably corresponds to the application force 112 (see Fig. 4) with which the microneedle array 102 is applied.

In the embodiments shown, the housing 16, i.e. the base 12 and the cover 14, the punch 18, and the microneedle array 102 as well as the openings 15, 17, 19 are essentially cylindrical having a circular cross-section. As such, the application device 10 and the application system 100, except for the patch 40, are generally cylindrical. Nevertheless, in other preferred (not shown) embodiments one or elements can have different shapes, for example rectangular or square shapes. Furthermore, it is preferably possible that the patch 40 has a circular shape.

Preferably, the opening 19 of the cover 14 has a cross-section smaller than the cross-section of the punch 18, such that the punch is secured within the housing 16, particularly the opening 17 in the base 12 against a movement out of the opening 17 in a direction opposite to the application direction 11.

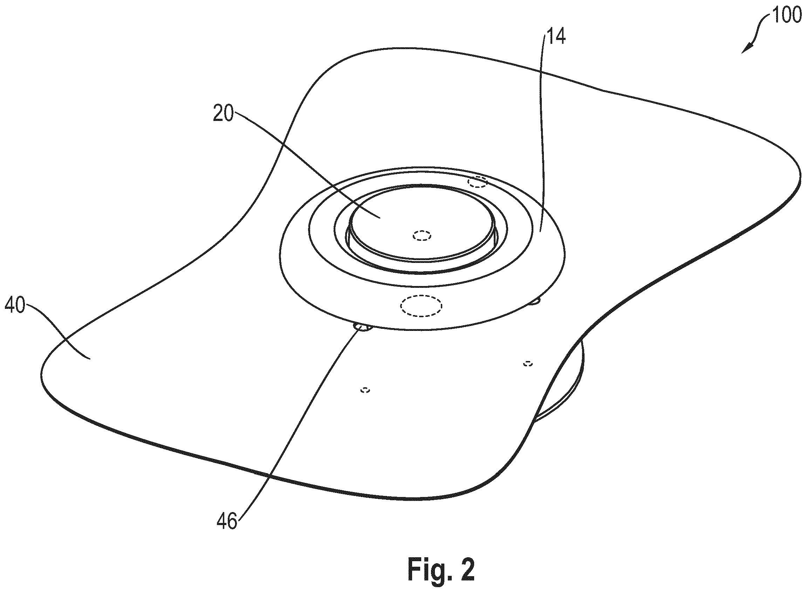

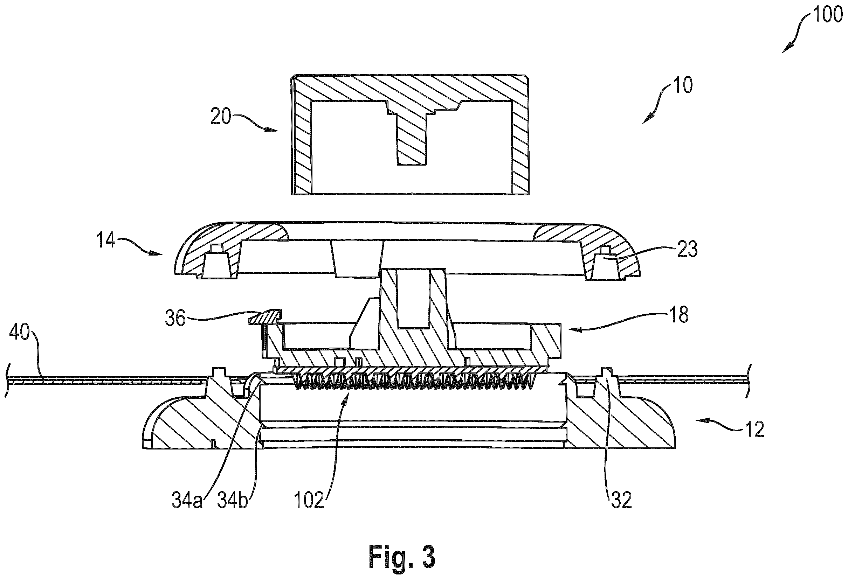

Figure 2 shows a perspective view of the application system 100 of Fig. 1. Figure 3 shows a sectional exploded view of the application system 100 of Fig. 1.

Figure 4 shows the application system 100 of Fig. 1 in a second state, the application state.

The punch 18 has been moved to the application position B due to the actuation of the user. The hooks 36 passed the protrusion 34b, thereby securing the punch 18 in the application position B. The microneedle array 102 is thus emerged from the housing 16 and can be applied with pressure into the skin (not shown) of a user. In the application position B, the punch 18 is moved out of the base, particularly from the skin surface 28. Thus, the punch 18 and/or the microneedle array 102 protrudes from the base 12, particularly from the skin surface 28, at least protrudes further compared to the starting position A.

Actuation is user who exerts a pressing force 110 on the button 20, which is transmitted to the microneedle array 102 via the punch 18 and thus causes the application force 112 to apply the microneedle array 102 into the skin. The skin of a user exerts a restoring force 114 against the application force, which forces the microneedles 104 of the microneedle array 102 out of the skin.

The patch 40 is preferably attached to the skin, preferably tensioned, for example due to a flexible and/or stretched adaptation of the patch 40, in the first state, i.e. when the punch 18 is in the starting position A. The patch 40, particularly when the punch 18 is in the application position B (as shown in Fig. 4), thus preferably provides a tension force 116, for example due to the stretching, driving the punch 18 onto the skin against the restoring force 114. The tension force 116 together with the application force 112 thus particularly provides a further, preferably lasting, pressing of the punch 18, such that the microneedle array 102 is applied into the skin over a time period with pressure.

Figure 5 shows a perspective exploded view of another application device 10 according to the invention. The embodiment of Fig. 5 is based on the embodiment of Fig. 1.

The base 12 of the application device 10 of Fig. 5 essentially corresponds to the base 12 of the embodiment of Fig. 1. The base 12 is connected to the cover 14 via pins 32, whereby the patch 40 is clamped between the base 12 and the cover 14 and fixed by the pins 32 passing through pre-cut openings 46 or piercing openings 46 through the patch 40.

A punch 18 is arranged movable within the opening 17 of the base 12 and can be arranged in a starting position A, whereby the deflectable hooks 36 passed the radial protrusion 34a.

The punch 18 can be displaced by a lever 50 when activated, i.e. when the lever 50 is folded over by a user. The lever 50 is pivotably mounted in pivot joints 60 of the cover 14 by means of two pivot pins 54 of the lever 50. When a user pivots the lever 50 by moving the lever 50 on the actuation end 53 of the lever 50, the pressing end 52 of the lever 50 directly contacts a pressing surface 62 of the punch 18 and thereby moves the punch 18, and preferably a microneedle array 102 connected to the punch 18 (not shown), in the application direction 11. As such, the punch 18 is moved into the application position B, whereby the hooks 36 pass the protrusion 34b to secure the punch 18 in the application position B against movement in the direction opposite the application direction 11.

It is preferred that the lever 50 comprises a visual marking visible, particularly exposed, when the punch 18 has been displaced from the starting position A into the application position B. For example, the surface 56 of the lever 50, which corresponds to the surface exposed in the starting state, i.e. when the punch is in the starting position A, can have a visual marking and/or the opposite surface 58 of the lever 50 which is the surface exposed in the application state, i.e. when the lever 50 is fully pivoted can have a visual marking. For example, the surface 56 can have a text and/or symbol indicating the starting state and/or a color area or is completely colored, for example in red or orange. Furthermore, the surface 58 can have a text and/or symbol indicating the application state and/or a color area or is completely colored, for example in green.

Additionally, particularly with the embodiment of Fig. 1 and/or of Fig. 5, the application device 10, particularly the latching devices 38a, 38b, can be adapted to provide an audible and/or tactile signal when the punch 18 reaches the application position B. The audible and/or tactile signal can be emitted by the latching device 38a, 38b. For example, if the hooks 36 snap due to deflection when passing the protrusions 34a, 34b and/or snaps back from deflection, the hooks 36 may emit an audible and/or tactile signal.

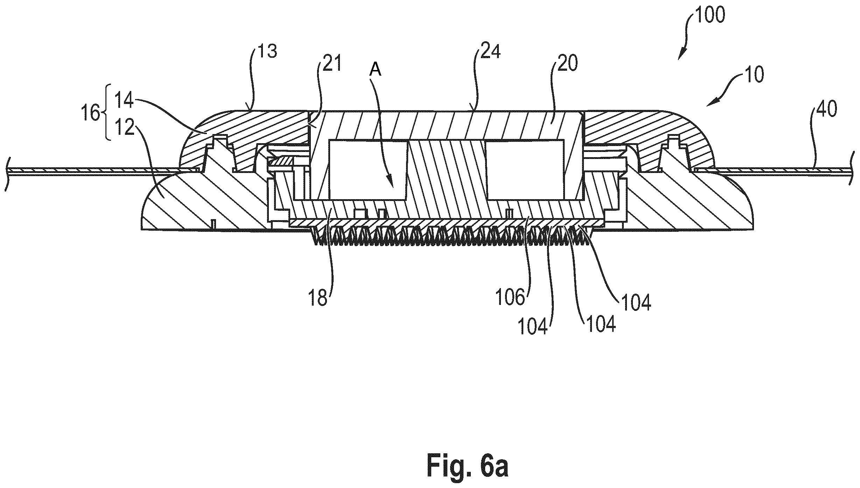

Figures 6a-6b show another preferred embodiment of the application system 100 according to the invention comprising another preferred embodiment of the application device 10 according to the invention.

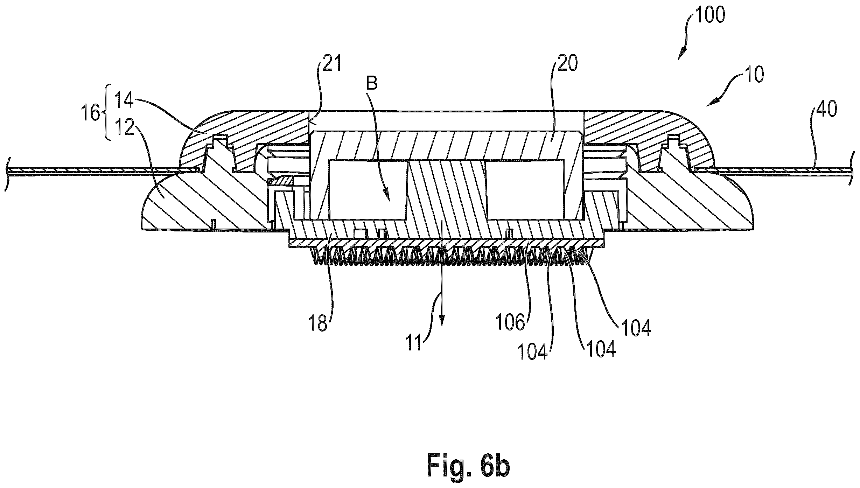

The embodiments of Figures 6a-6b are based on the embodiment of Figure 1. Figure 6a shows the state, the starting state, in which the punch 18 is in the start position A and Figure 6b shows the state, the application state, in which the punch 18 is in the application position B.

In contrast to Figure 1, in the embodiments of Figures 6a-6b, the button 20 is flush with the housing 16 in the starting state (see Figure 6a). This flush arrangement is preferably implemented by arranging the button 20, particularly the application surface 24 of the button 20, flush with the cover 14, particularly a top surface 13 of the cover 14.

After a movement of the punch 18 from the starting position A to the application position B (as shown in Figure 6b) due to an application actuation, particularly by a user pressing the button 20, the button 20 is arranged set back inwards to the top surface 13. In this position, an inner lateral surface 21 of the cover 14 is visible from the outside. This inner lateral surface 21 preferably comprises a visual marking, such as a symbol and/or a color, preferably green, for example, the inner lateral surface 21 can be colored, such that a user can identify that the application position B is reached by observing the visual marking. Preferably the visual marking is only visible if the punch 18 reaches the application position B. It is preferred that the visual marking is not visible, preferably covered, in the starting state, particularly the visual marking on the inner lateral surface 21 is covered by the button 20 in the starting state.

Furthermore, in contrast to Figure 1, in the embodiments of Figures 6a-6b, the button 20 and punch 18 are connected to each other by a flat contact, in particular they are materially bonded, for example glued and/or welded to each other, or they are integrally connected, particularly as one piece.

In Figures 1 and 6a, the microneedle array 102, particularly the microneedles 104, is arranged to protrude, at least partly, out of the opening 17 in the starting state and is thus exposed in the starting state. Alternatively, in other preferred embodiments (not shown), particularly based on the embodiments of Figures 1 and/or 6a, the application device 10 can be adapted to arrange the microneedle array 102, particularly the microneedles 104, is set back within the opening 17 in the starting position, preferably so that the microneedles 104 do not protrude in the start position. In the illustrated embodiment of Figures 1, 4, 6a and 6b, there is no opposing hook 36 shown directly opposite the hook 36 on punch 18. In a preferred embodiment, such an opposing hook 36 can be provided (see Figure 5).

## Claims

1. An application device (10) for microneedles, comprising:
a housing (16) comprising a base (12) adapted to be arranged on the skin,
a punch (18) arranged inside the housing (16), wherein the punch (18) is movable inside the housing (16) in an application direction (11) from a starting position (A) to an application position (B) to apply an application force (112) to a microneedle array (102) for applying the microneedle array (102) into the skin,
wherein in the starting position (A) the punch (18) is secured against movement in the direction opposite to the application direction (11).

2. The application device (10) according to claim 1, wherein the application device (10) comprises a latching device (38a, 38b) between the housing (16) and the punch (18) to secure the punch (18) in the starting position (A) and/or in the application position (B) against a movement in the direction opposite to the application direction (11).

3. The application device (10) according to claim 2, wherein the latching device (38a, 38b) comprises a one-way lock to prohibit movement of the punch (18) in the direction opposite to the application direction (11).

4. The application device (10) according to claim 2 or 3, wherein the latching device (38a, 38b) is adapted to allow free, in particular unimpeded, movement of the punch (18) from the starting position (A) and/or the application position (B) in the application direction (11).

5. The application device (10) according to any one of claims 1-4, wherein the application device (10) comprises an, preferably adhesive, patch to attach the base (12) to the skin.

6. The application device (10) according to claim 5, wherein the patch is adapted to tension the base (12) against the skin, in particular against a restoring force (114) exerted via the punch (18).

7. The application device (10) according to any one of claims 1-6, wherein the housing (16) comprises a cover (14) connected to the base (12) closing the base (12) on a side opposite a skin surface of the base (12).

8. The application device (10) according to claim 7, wherein the patch is fixed, particularly pierced, between the cover (14) and the base (12).

9. The application device (10) according to any one of claims 1-8, wherein the application device (10) comprises an operation device, particularly a lever (50) or a button (20), acting on the punch (18) to transfer a force (110, 116) to the punch (18) corresponding to the application force (112).

10. The application device (10) according to any one of claims 1-9, wherein the application device (10) comprises a visual marking visible, particularly exposed, when the punch (18) has been displaced from the starting position (A), preferably into the application position (B).

11. A microneedle application system, comprising:
- the application device (10) of any one of claims 1-10, and
- a microneedle array (102) connected to the punch (18) via a material-bonded connection and/or a form-fit; or integrally.

12. A method for manufacturing a microneedle application system according to claim 11,
- manufacturing of a microneedle array (102) in a mold,
- providing, in particular manufacturing, a housing (16) and a punch (18); and
- arranging the punch movable inside the housing (16).

13. The method according to claim 12, wherein the method comprises the further step of:
- demolding the microneedle array (102) from the mold by the punch (18).

14. The method according to claim 12 or 13, wherein the method comprises the further step of:
- connecting a patch (40) to the housing (16), particularly arranging the patch (40) between a base (12) and a cover (14) of the housing (16).

15. The method according to any one of claims 12-14, wherein the step of arranging the punch movable inside the housing (16) comprises:
- inserting the punch (18) into the housing (16) into a starting position (A) by passing a latching device (38a) between the housing (16) and the punch (18) to secure the punch (18) in the starting position (A) against a movement in the direction opposite to an application direction (11) of the movement of the punch (18).
